# EUROPEAN PATENT SPECIFICATION

(11) **EP 2 580 337 B1**
(45) Date of publication and mention of the grant of the patent: **02.08.2023**
(21) Application number: 11726925.8
(22) Date of filing: 10.06.2011
(51) Int. Cl.: C12N 15/89, C12M 3/00, G02B 3/00

(54) **OPTICAL TRANSFECTION**
OPTISCHE TRANSFEKTION
TRANSFECTION OPTIQUE

(30) Priority: 11.06.2010 GB 201009800
(43) Date of publication of application: 17.04.2013
(73) Proprietor: University Court of the University of St. Andrews, St. Andrews KY16 9AJ (GB)
(72) Inventor: DHOLAKIA, Kishan, Fife KY 16 9SS (GB); MA, Nan, Fife KY 16 9SS (GB); ASHOK, Praveen Cheriyan, Fife KY 16 9SS (GB); STEVENSON, David, Fife KY 16 9SS (GB); GUNN-MOORE, Francis James, St. Andrews KY16 9TF (GB)
(74) Representative: Marks & Clerk LLP
(86) International application number: PCT/GB2011/000881
(87) International publication number: WO 2011/154710

(56) References cited:
- EP-A1- 0 260 742
- EP-A1- 0 391 558
- EP-A1- 1 225 221
- EP-A1- 1 607 474
- DE-A1- 3 707 111
- US-A- 4 338 352
- US-B1- 6 488 414
- X. TSAMPOULA ET AL: "Fibre based cellular transfection", OPTICS EXPRESS, vol. 16, no. 21, 13 October 2008 (2008-10-13), page 17007, XP55645466, US ISSN: 1094-4087, DOI: 10.1364/OE.16.017007

## Description

### Field of the invention

The present invention relates to a fibre based optical transfection system.

### Background of the invention

The introduction of therapeutic and other agents into cells, which are otherwise membrane impermeable, remains a key requirement in cell biology. Currently, a variety of transfection methods are used to solve this problem, including chemical, physical, optical, electrical, and viral. Optical transfection offers selectivity, specificity, high transfection efficiency and good post-transfection cell viability. By applying a tightly focused laser beam on the cell membrane, optical transfection can transiently and locally increase the permeability of the cell's plasma membrane to allow for example nucleic acids to be internalized.

Most optical transfection techniques that have been used employ free space (bulky) optical setups which limit the potential application of the technology for *in-vivo* experiments. In addition, the transfection efficiency achieved is highly dependent on the quality of the photoporation beam, so expertise in optical alignment is necessary to achieve efficient transfection.

Fibre based femtosecond optical transfection has been proposed. This uses an axicon tipped optical fibre for light delivery. The axicon tip is made using hydrogen fluoride based etching, which makes the fabrication hazardous. Also the transfection efficiency is very sensitive to the quality of the axicon tip. In addition, the short working distance produced by the axicon makes the targeting of the beam focus at the cell membrane very difficult: particular care has to be taken to make sure both fibre tip and cells are not damaged.

Microlensed fibres are widely used in the field of communication for increasing coupling efficiency between terminals and interconnect. Various fabrication procedures are reported for the fabrication of microlensed fibres. Melting the fibre tip by an electric arc discharge or heating to form a lens are the most widely used methods to fabricate a communication standard microlensed fibre. However, these methods do not provide high reproducibility and only lenses with a comparatively large radius of curvature can be fabricated. Polishing can be used to make axicon lenses of different angles; however, this is complex, time consuming and expensive. Femtosecond two-photon lithography is a highly flexible technique, in which micro-structures are directly inscribed on surfaces point by point. However, this technology is in its infancy and the manufacturing cost is unacceptably high for practical applications. Other indirect fabrication methods use coreless silica fibre, micro-silica spheres or a combination of these. All these procedures have disadvantages such as complexity, high cost or lack of flexibility.

DE 37 07 111 A1 teaches a method for inserting genetic material into organelles. Organelles suspended in a buffer solution are picked up in a fine glass capillary and treated by focused laser beams, so that an opening is briefly produced in the membrane coat. The foreign genetic material in the buffer solution can diffuse into the organelle through the opening.

EP 0 391 558 A1 teaches a method of making an optical fibre. An optical fibre having a free end is contacted with a curable material. On curing, the material forms a member having light-scattering properties on the free end.

US 4 338 352 A teaches a process for producing a guided wave lens on optical fibres. A lens is produced on the end of a single mode optical fibre by first coupling light into the fibre so that the light projects out the end of the fibre. The fibre end is then dipped in and out of a drop of negative photoresist and held in a vertical position. The photoresist wicks up the fibre and dries. The dipping and drying is continued until a sufficient lens of photoresist is built up.

EP 1 607 474 A1 teaches an apparatus and method for injecting a substance into a cell. A surface of a cell is irradiated with a laser beam to make an opening in the cell membrane and a drug fluid is inserted into the cell through the opening.

EP 1 225 221 A1 teaches a laser-based method for cell wall and membrane perforation. A cell or a living tissue is irradiated with a laser beam through an optical fibre.

X. Tsampoula et al., Optic Express, vol. 16, no. 21, pages 17007-17013, describe a system for cell transfection using an optical fibre provided with a conical lens (axicon) on the fibre facet. The lens is obtained by chemical etching.

### Summary of the invention

According to the present invention there is provided a system according to claim 1. The system may be used for delivery of a material, for example a drug or DNA, into a cell. The system may include a delivery tube or channel for localized delivery of the material to the cell. Preferably, the optical fibre is positioned in the tube or channel. The delivery tube or channel may be a microcapilliary.

The system may include a laser, for example at least one of: femtosecond laser, nano-second laser, pico-second laser and continuous wave laser. The laser is provided for optically porating a sample, for example a cell.

The system may include a multimode fibre for delivering an illumination beam for illuminating a sample area, so that the sample being treated can be viewed during and/or after treatment.

A method for fabricating a microlens on a fibre is described herein which comprises using an optically curable material, for example an ultraviolet (UV) curable adhesive. The lens characteristics can be tailored by changing the parameters of curing the adhesive. Using this technique microlenses yielding a very small focal spot (2-3 µm) at a relatively large working distance (~15 µm) can be made.

The method involves applying an optically curable material to an end of the fibre and exposing the end of the fibre to a laser radiation suitable for curing the material. Depending on the profile of the curing beam, different types of micro-lenses having potentially different applications can be made. The fabrication procedure is simple and requires only basic focusing optics, and relatively simple, low power lasers, for example a blue diode laser, to achieve preferential and controlled curing of the optical carable material. The beam shape of the curing laser beam may be modified to obtain a wide variety of microlenses with different shapes and parameters.

The method may involve removing uncured material remaining after exposure to the curing radiation. The method may also involve varying curing exposure time, curing exposure rate, alignment of the fibre tip with respect to the curing beam for obtaining different types of microlens.

The method may involve forming a drop of the liquid form of uncured, but optically curable, material on the end of the fibre. The method may involve dipping the end of the fibre into the optically curable material so that some of the optically curable material adheres to the end of the fibre.

The optically curable material may be sensitive to UV radiation and/or may be an adhesive.

The microlens may be shaped to tightly focus, collimate or cause divergence of the output from the optical fibre.

### Brief description of the drawings

Various aspects of the present invention will now be described by way of example only and with reference to the accompanying drawings, of which:
Figure 1(a) is an image of an integrated fibre based optical transfection device;
Figure 1(b) is an expanded view of part of the device of Figure 1(a);
Figure 1(c) is another expanded view of a part of the device of Figure 1(a);
Figure 2 is a schematic view of a fibre based optical transfection system that includes the fibre based optical transfection device of Figure 1;
Figure 3 shows images of cells recorded using the system of Figure 2;
Figure 4 is an image of successfully transfected cells (expressing a red fluorescent protein);
Figure 5 is a plot showing transfection efficiency of CHO-K1 cells achieved using an axicon tipped fibre, a microlens tipped fibre and the system of Figure 2, respectively, as well as transfection efficiency of HEK-293, also measured using the system of Figure 2;
Figure 6 is a schematic view of a system for fabricating a microlens;
Figure 7 illustrates various steps in a method for making a microlens tipped fibre;
Figure 8 is an SEM image of a microlens formed using the method of Figure 7;
Figure 9 shows the results of a ray trace model for a lens made using the method of Figure 7, and
Figures 10(a)-10(c) showexamples of different curing beams and Figures 10(d)-10(f) show examples of the resulting structures which can be made by changing the curing beam distribution in the method of Figure 7 but which are not part of the invention;
Figure 11(a) shows an example of a collimated curing beam;
Figure 11(b) shows an image of a lens formed using the collimated curing beam of Figure 11(a) which is not part of the invention; and
Figure 11(c) shows a ray diagram corresponding to the lens of Figure 11(b).

### Detailed description of the drawings

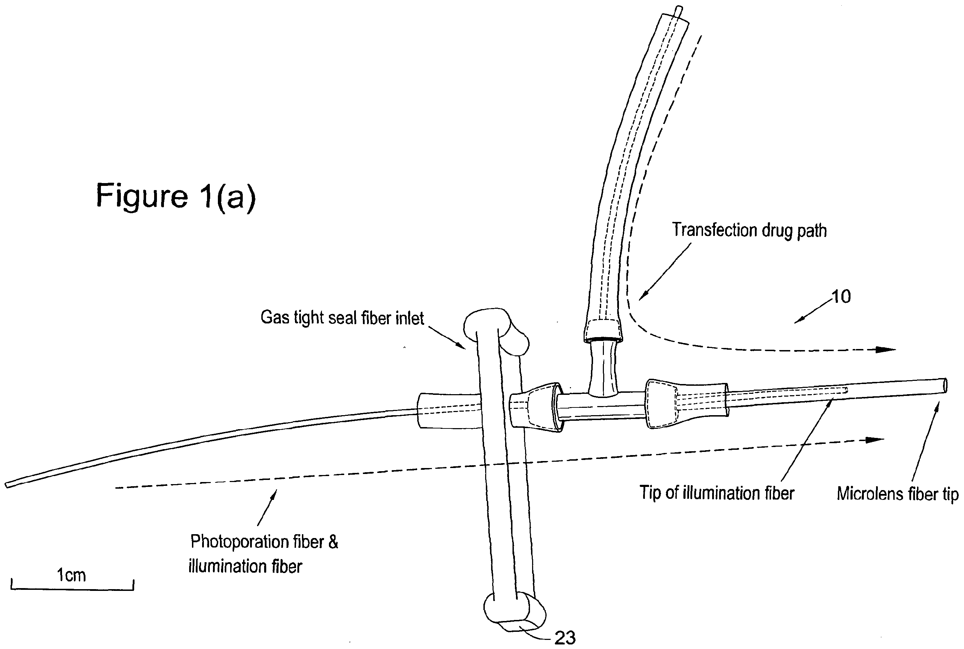

Figures 1(a) to (c) show an intergrated fibre based optical transfection device 10. This has a fibre with a lens formed at its tip (microlensed fibre) 12 for delivering a focussed laser beam near a cell membrane. A technique for forming the lens on the end of the fibre will be described in more detail later. To achieve localized drug delivery during optical transfection, the microlensed fibre 12 is integrated within a capillary tube 14, which is used to deliver fluid containing the material that is to be transfected to the cell. The micro-capillary 14 is attached to a first port 16 of a barbed three port T connector 18 (Harvard Apparatus, 72-1487) using flexible plastic tubing 20 (for example Tygon T3601, inner diameter =0.8 mm, outer diameter ₌ 2.4 mm). The microlensed fibre 12 is inserted through the second port 20, into the micro-capillary 14 (inner diameter ₌ 0.58mm, outer diameter ₌ 1mm) and the tip of the fibre 12 positioned close to the tip of the micro-capillary 14. A cleaved multimode fibre 22 of core diameter 200 µm (Thorlabs, BFL37-200) is inserted into the micro-capillary 14 for illuminating the sample. The optimum distance of the tip of the multimode fibre 22 from the apex of the microlens was experimentally estimated to be ~1 cm from the tip of microlens tipped fibre to achieve the best contrast for the sample. A slide clamp 23 (WPI, Luer Valve Assortment, 14042) was used to seal the flexible tubing, attached to the fibre inlet port, in order to ensure that the device 10 was airtight during sample injection. Flexible tubing 24 is attached to the third port 26 for delivery of the material to be transfected, for example DNA.

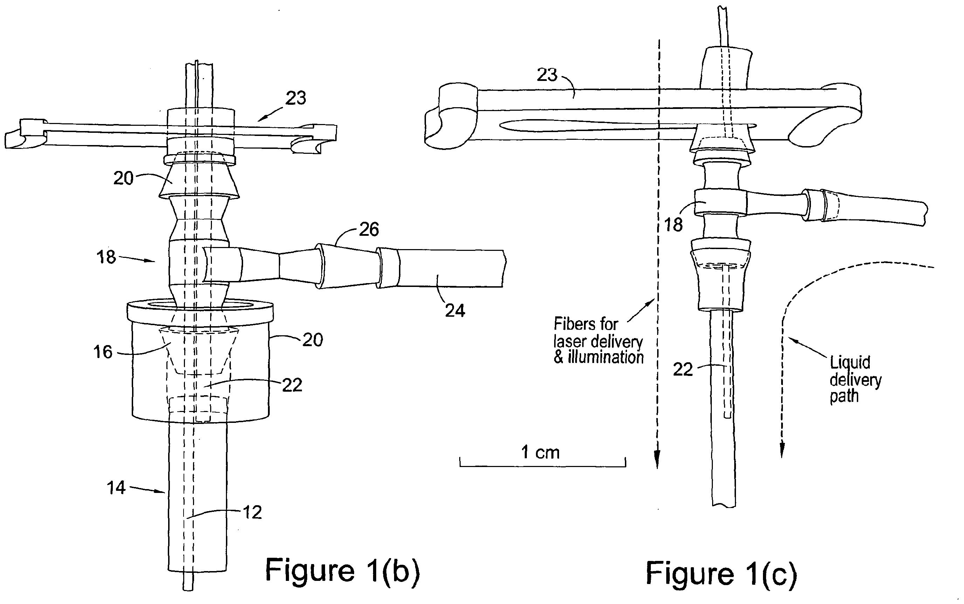

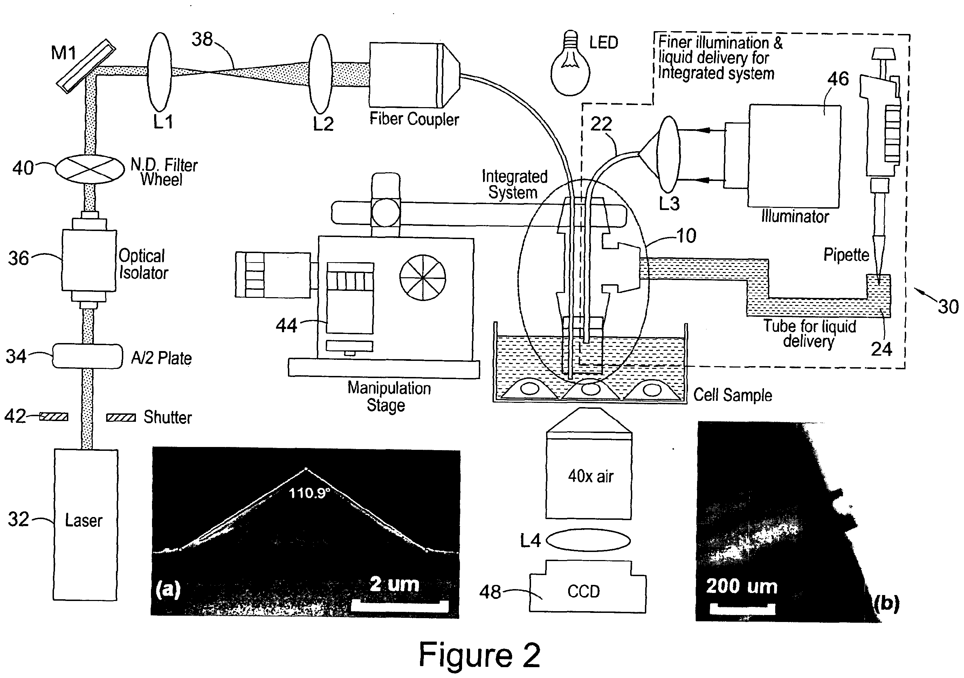

Figure 2 shows a photoporation system 30 that can be used with the integrated device 10 of Figure 1. A laser 32, for example a Ti: Sapphire laser, is used to form a collimated poration laser beam, which is directed into a combination of a half wave plate 34 and an optical isolator 36 (Laser2000, UK, I-80-2) to eliminate back reflection from the beam path. A magnifying telescope 38 (typical magnification 1.6X) is used to expand the incoming laser beam, which is coupled to the microlensed optical fibre 12 through a fibre collimator (Thorlabs, F810FC-780). The fibre output power is adjusted using a variable neutral density (ND) filter wheel 40 appropriately placed in the beam path. During photoporation collimated light from the laser 32 is focused by the microlensed fibre 12 onto the cell membrane. The average power of the beam is kept at 20 mW, with a peak power per pulse of 0.24 kW. A mechanical shutter 42 is used to control the time duration of the laser dosage on the cell membrane. The integrated transfection device 10 used for poration is mounted on a three axis (X-Y-Z) translation stage 44 for insertion into the sample medium. An illuminator 46 is provided for illuminating the sample using the illumination fibre. Liquid containing the material that is to be transfected is delivered using the liquid delivery tube 24 connected to the micro-capillary 14. Below the sample area is provided a camera 48 for caputring images of the cells before, during and after transfaction.

A sample including cells that were to be porated was put in a petri dish. To transfect material into the sample cells, poration was instigated by the laser emitting at 800 nm, with output pulse duration of -100 fs and a pulse repetition frequency of 80 MHz (Coherent, MIRA). For the purposes of comparison, this was done for both an axicon tipped fibre (as known in the prior art) and a microlens tipped fibre. At the output end of both the microlens and axicon tipped fibres, the pulses undergo stretching due to a nonlinear phenomena occurring inside the fibre - self-phase modulation (SPM) and group velocity dispersion (GVD) - giving an overall pulse duration of approximately 800 fs. Axicon tipped fibre transfection was performed as described previously. For microlens tipped fibre transfection, due to restrictions imposed by the geometry of the fibre and the imaging path, the fibre was tilted at ~ 5 - 10° with respect to the vertical axis. With a white LED light source on top and an imaging system below the sample, the sample cells were observed during the transfection procedure using the camera below the sample.

Two cell samples were tested in the system of Figure 2. These were CHO-K1 and HEK-293. The cells were cultured in modified eagles medium (MEM) containing 10% foetal calf serum (FCS), 18 IU/ml of penicillin, 18 µg/ml of streptomycin, 1.8 mM of L-Glutamine ("complete medium") in a humidified atmosphere of 5% CO2 / 95% air at 37 °C. Cells were grown to sub-confluence in 30 mm diameter glass-bottomed culture dishes (World Precision Instruments, Stevenage, UK) in 2 ml of culturing cell media (MEM). Prior to experimentation, the cell monolayer was washed twice with OptiMEM (Invitrogen) and for all experiments (except the integrated system where the solution was delivered microfluidically), the sample was bathed in 1 ml solution of OptiMEM containing 9 µg/ml mitoDsRED plasmid, encoding a mitochondrially targeted Discoideum Red Fluorescent protein (BD Biosciences, Oxford, UK).

To ensure the sterility of the drug delivery system, before each transfection experiment 2 ml of 70% ethanol was run through to sterilize the whole system and was subsequently dried using filtered air. The capillary tube was tested for multiple transfection experiments and the cell viability for subsequent experiments showed that the system remains sterile with the above mentioned sterilization procedure.

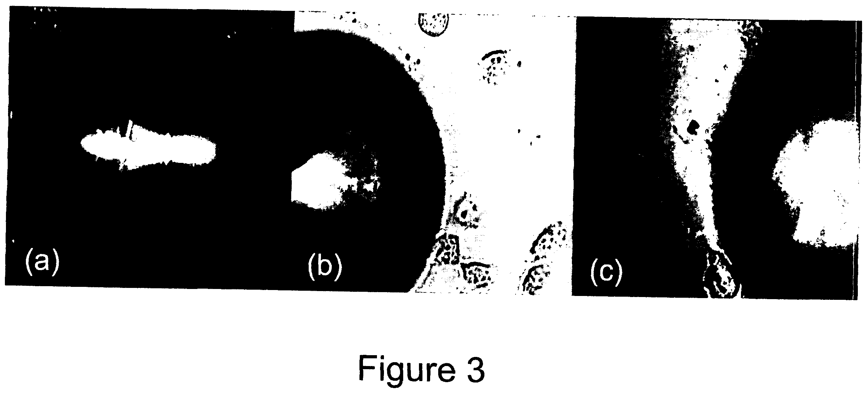

The pipette loaded with sample was connected to the capillary tube of the integrated system. Controlled injection of DNA locally into CHO-K1 and HEK-293 was achieved using the pipette during optical transfection. An image of cells recorded during optical transfection with the integrated illumination system, is shown in Figure 3. Despite the poor image contrast due to a shadow cast by the photoporation fibre, when targeted cells were imaged the cell boundaries were visible, which permitted them to be transfected.

In order to monitor potentially spontaneous transfected cells, each photoporated sample dish was accompanied by a control sample dish in which cells were cultured, bathed in plasmid DNA solution and then experienced the fibre presence in the absence of laser radiation. Experimental details of the number of treated cells and the results are shown in Table. 1. The number of spontaneously transfected cells varied between 0 - 2 cells for each sample dish.

**Table 1**

| | *Cell type* | *No of Dish treated* | *Total No. of treated cells* | *Transfection Efficiency (±SEM) (%)* |
|---|---|---|---|---|
| Axicon tipped fibre | CHO-KL | 15 | 450 | 30.22 ± 5.36 |
| Microlens tipped fibre | CHO-KL | 20 | 800 | 40.25 ± 3.39 |
| Integrated system | CHO-KL | 15 | 525 | 45.71 ± 4.84 |
| | HEK-293 | 5 | 175 | 64.00 ± 4.10 |

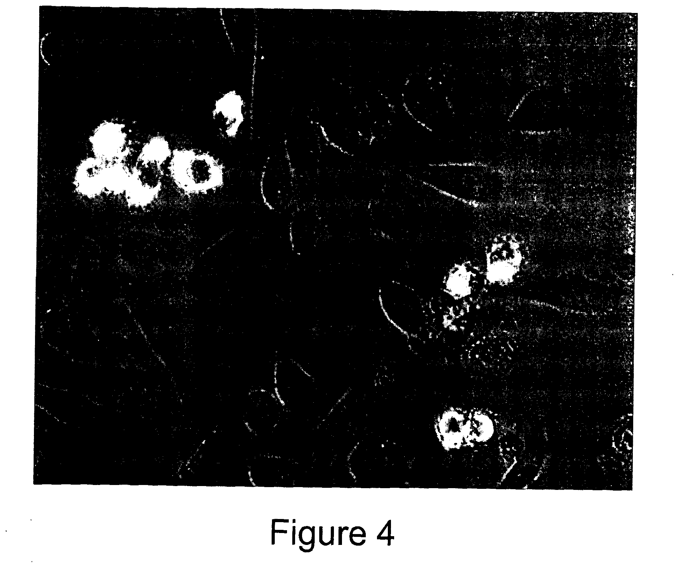

During laser irradiation no visual response was observed. After the laser treatment, the cell monolayer was bathed in complete medium and returned to the incubator. The sample was viewed forty eight hours later under a fluorescent microscope, where successfully transfected cells expressed the red fluorescent protein as shown in Figure 4.

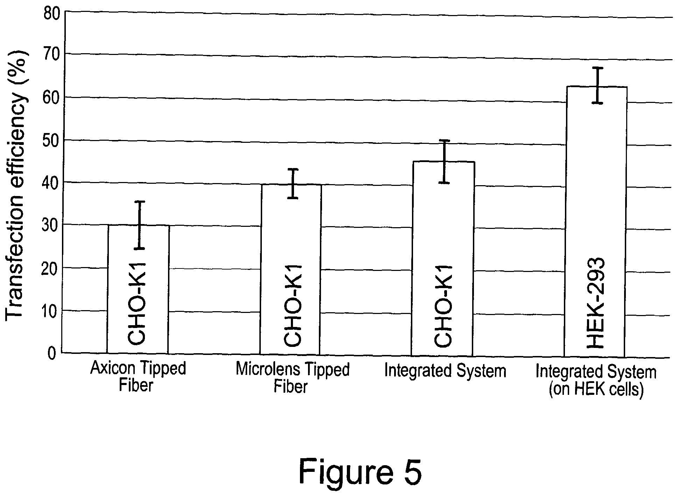

Figure 5 shows a comparison of the transfection efficiency of CHO-K1 cells achieved using an axicon tipped fibre, a simple microlens tipped fibre (no localised fluid delivery) and the integrated device 10 of Figures 1 and 2 respectively. In addition, Figure 5 shows the transfection efficiency of HEK-293 when treated with the integrated device 10. The transfection efficiency is defined as the number of cells expressing the correctly targeted red fluorescent protein 48 hours after laser treatment divided by the total number of cells that were laser treated in a particular area of interest.

From Figure 5, it can be seen that the efficiency of the fibre based optical transfection technique of the present invention is comparable to that of free space transfection. Also, the microlens tipped fibre provides a higher transfection efficiency and smaller standard deviation in efficiency, than the axicon tipped fibre method. This reflects the fact that the longer working distance makes the manipulation of a microlens tipped fibre easier and more stable compared to an axicon tipped fibre. During the transfection procedure, the axial focal position needed to be found only at the beginning of the procedure and then multiple cells in the same sample dish could be photoporated just by moving the fibre mount laterally. This causes less damage to cells and the fibre tip, high transfection efficiency and more consistency.

Using the integrated device 10 of the invention, highly localized delivery of DNA-containing fluid can be achieved. The long working distance lens allows easy manipulation of the fibre tip over the sample and hence better throughput for photoporation compared to its axicon tipped counterpart. The localised drug delivery makes the technology amenable to single cell studies. Cell boundaries can be observed during transfection using a multimode fibre based illumination system embedded into the integrated system. Using microlensed fibre with an integrated delivery channel opens up prospects for a portable "hand-held" system that can locally deliver therapeutic agents and transfect cells within a fibre geometry placing minimal requirements upon any microscope system.

Another aspect of this disclosure provides a method for making a fibre with a microlens on its tip using a UV curable adhesive. To demonstrate the effectiveness of this technique a lens was fabricated on a commercially available single mode fibre. This has a mode field diameter of 5.6 µm, cladding diameter 125 µm, and an operating wavelength of 830±100 nm (Thorlabs, SM800-5.6-125). A UV curable adhesive (Norland, NOA 65) with optimum sensitivity for curing in the 350 - 380 nm range was used due to its good adhesion to glass, fast curing time, easy processing, suitable refractive index (1.524 for polymerized resin) and high transmission efficiency (-98%) at 800 nm. These characteristics make the polymer lens ideal for the delivery of high peak power pulsed laser light without damaging the structure. The UV curable adhesive was cured with a laser beam specially shaped to obtain a desired shape of the lens.

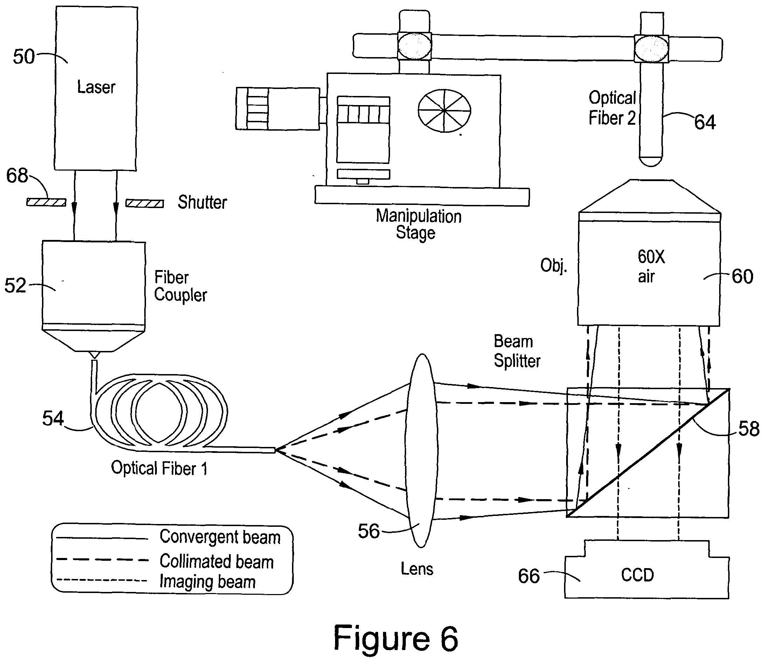

Figure 6 shows a set up for fabricating a microlens using free space optics for directing a laser beam onto the end of an optical fibre. This has a violet diode laser 50 (405 nm) coupled through an objective 52 (x10, Newport, UK) into a single mode optical fibre 54 (Thorlabs, S405-HP) with a coupling efficiency of -45%, in order to improve the lateral profile of the laser beam. The end of the fibre is positioned at the focal point of a lens 56, which collimates light from the laser and directs it onto a beam splitter 58. The lateral beam profile of the output beam is measured using a long working distance objective (Mitutoyo x100 infinity-corrected, WD ₌ 6mm) to confirm a high quality TEM₀₀ Gaussian beam profile. The laser beam is then directed to an objective 60 (×60 Nikon) which focuses the light to a fabrication area, where a fibre 62 that is to be processed is vertically mounted on a xyz translation stage 64. A CCD camera 66 (WAT-250D) is provided to allow the tip of the fibre to be imaged during the curing process. Exposure time is controlled using a shutter 68 (Newport, UK, model 845HP-02) at the output of the laser 50.

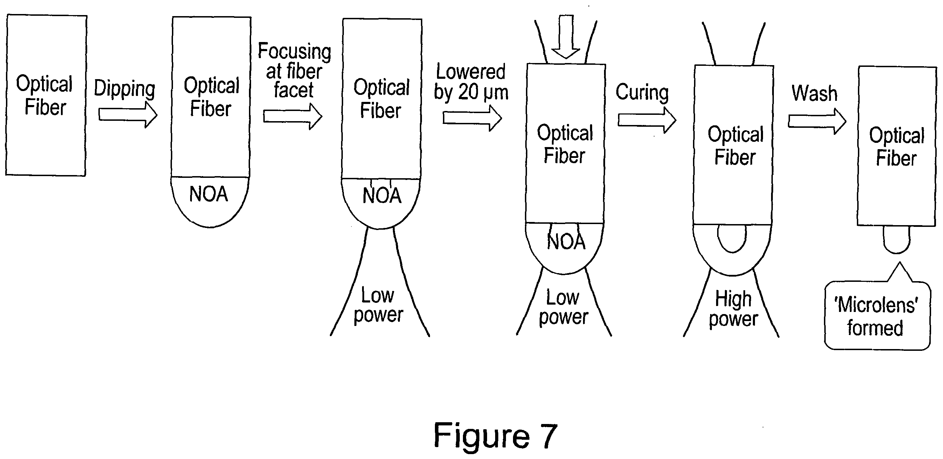

Figure 7 shows the steps involved in microlens fabrication. A well-cleaved optical fibre is vertically dipped and raised from a drop of UV curable adhesive such that a hemisphere of adhesive on the fibre tip is formed. The fibre is then mounted to the curing setup of Figure 6. Keeping the power of the laser lower than the threshold (<0.1 mW), below which curing process would not be initiated, the laser beam was positioned at the centre of fibre tip facet and defocused by 20 µm from the tip in order to get correct beam shape to produce the desired lens structure. In the example shown, the beam has a guassian profile, and the fibre and the beam are positioned, so that the beam waist is located in the region of the fibre tip. The power of the laser is increased to 0.5mW to start the curing process. At the beginning of the polymerization process, the UV adhesive partially cures around the centre of fibre facet followed by a growth towards the direction of the laser. After exposure for 5s, the un-polymerized adhesive was removed using acetone and a 'micro-stick' is created. At the apex of 'micro-stick' a curved facet is formed which acts as a focusing surface for the output beam from the fibre.

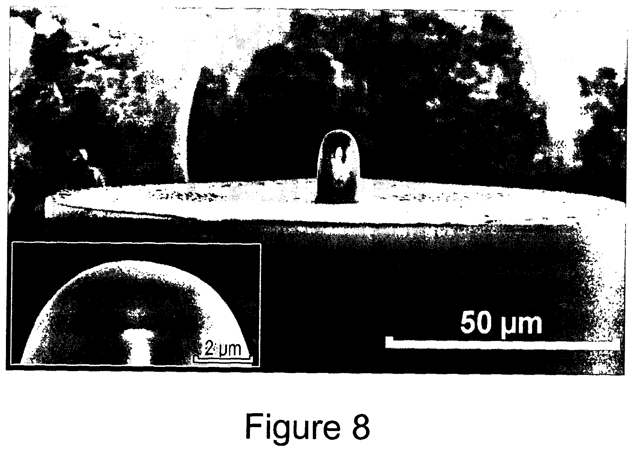

Figure 8 shows an SEM image of a microlens formed using this technique. The physical parameters of the lens were estimated from the SEM images. The height of the lens was estimated as 11 ± 1µm, base diameter 7 ± 1µm and top diameter 5 ± 1µm. An 800 nm laser from a Ti-Sapphire laser (Coherent, MIRA) was coupled to the microlens tipped fibre and the output beam from microlens was profiled in water from a series of lateral cross-sections with a 5 µm step change using a water immersion objective (x60 Olympus UPlanSApo) in conjunction with a CCD camera (WAT-250D). The working distance of the lens (distance of the focal plane from the apex of the lens) and the diameter of the focal spot were estimated from the beam. The estimated working distance of the beam was 15 ± 5 µm, focal spot diameter was 3 ± 0.05 µm and the beam divergence was 15°.

A ray tracing model of the lens fabricated at the tip of the fibre was built using optical design software (Zemax Development Corporation) from the parameters estimated from the SEM images and beam profiling. In the Zemax model, a radial source with a Gaussian profile was defined at a wavelength of 800 nm, which propagates from a cylinder of refractive index similar to that of the core of the fibre used. The output beam from the cylinder had same numerical aperture (NA ₌ 0.12) and mode field diameter (MFD = 5.6µm), as was defined by the specifications of the single mode fibre used for the experiments. A microlens was defined at the surface of the cylinder with a material of refractive index same as that of cured UV adhesive (Norland 65 - refractive index ~1.52). The lens was designed with physical dimensions estimated from the SEM image, keeping the radius of curvature of the surface close to the surface of the cylinder as 0 and the radius of curvature of the second surface (apex of the microlens) as a variable. The whole system was immersed in water and the radius of curvature of the lens was estimated which provided the experimentally measured working distance.

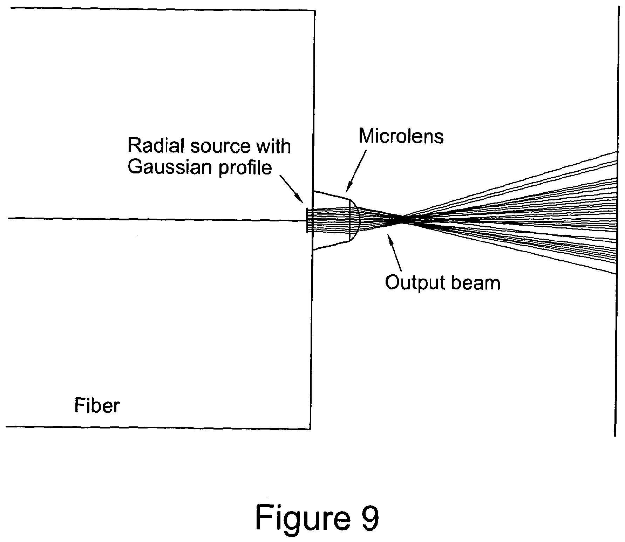

Figure 9 shows the results of the ray trace model. From this, it can be seen that the radius of curvature was estimated to be 7 µm ± 0.5 µm. With all the estimated parameters, beam profiling was performed on the model at a step size change of 5 µm and the diameter of the focal spot (2.9 µm) and divergence (16°) were calculated. These were found to be comparable with the experimental values.

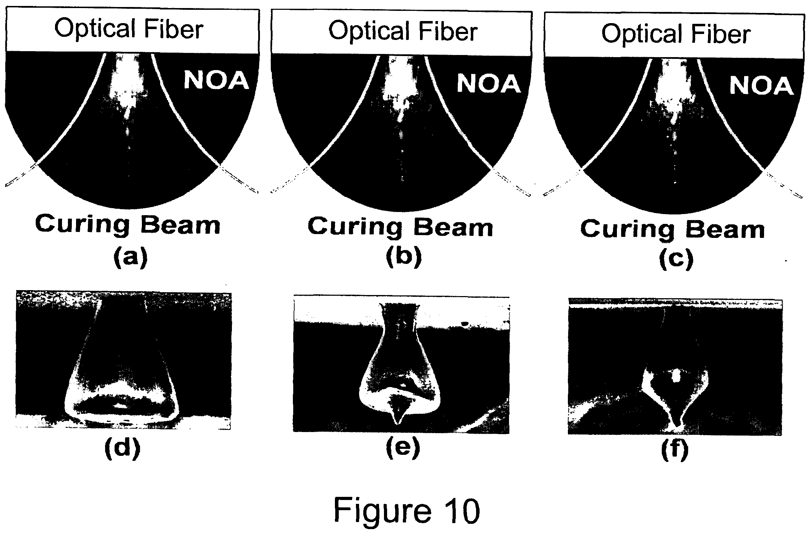

The UV curing fabrication procedure is highly flexible. By changing parameters such the light distribution near to the focus of the curing beam, intensity of the curing beam or curing time, it is possible to fabricate different structures at the tip of the fibre. Figure 10 shows examples of different structures which can be made by changing the curing beam distribution but which do not form part of the invention. Using curing beam (a), (b) and (c) with appropriate laser power and curing time structures like (d), (e) and (f) respectively were fabricated. As shown in Figure 10, the structures created are strongly related to the beam distribution.

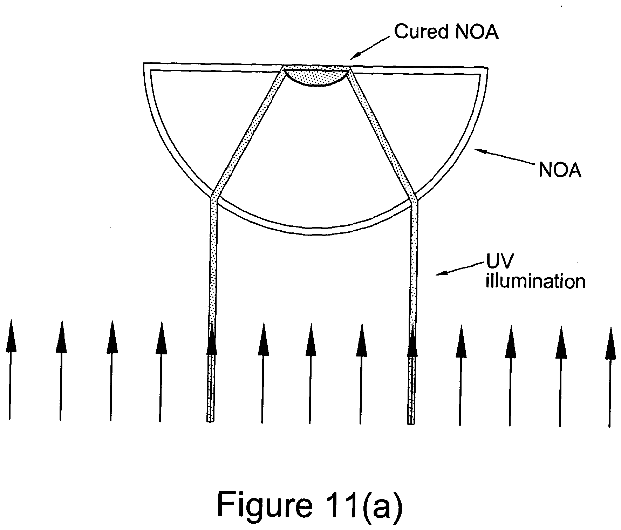

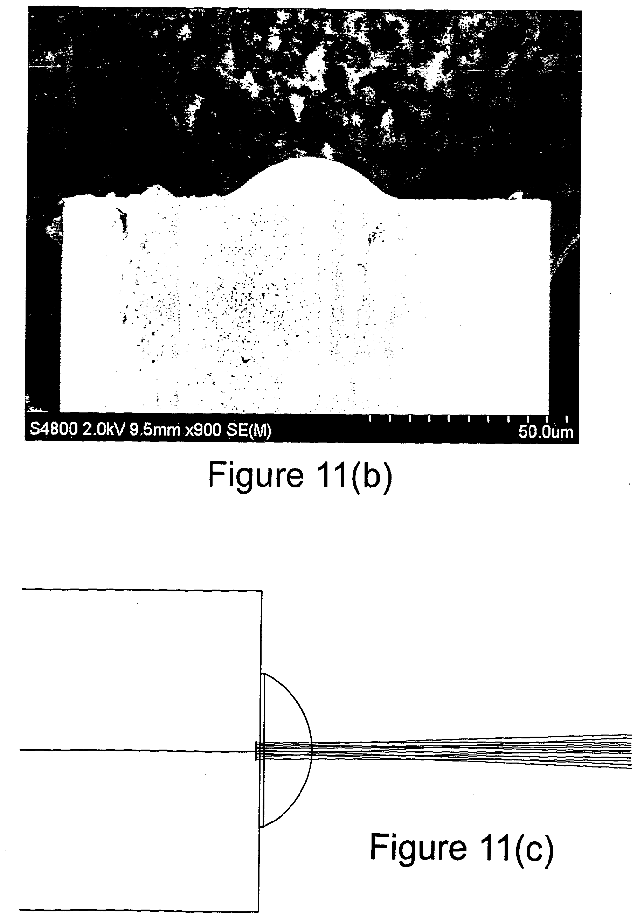

Whilst the description has focused on a fibre lens fabricated using a beam with a guassian profile, other curing beam profiles can be used. Figure 11(a) shows an example of a collimated curing beam which does not form part of the invention. In this case, the collimated beam is directed onto the hemispherical drop of curable material and focussed towards the tip of the fibre. This causes a smoothly curved lens to form. This fabrication does not require any intricate alignment of the curing beam. To test this, technique a piece of well cleaved single mode commercially available optical fibre was dipped into UV glue (e.g. Norland optical adhesive) vertically and pulled out slowly. The surface tension of UV glue allowed a hemisphere to be formed on the end of fibre tip. Then a collimated mercury lamp illumination was used to cure UV glue hemisphere. Due to the self-focusing property of hemisphere, the curing beam focused around the centre of the end of fibre tip, and so polymerization started from there and grew outward. The curing power and time determined the size of microlens and so has to be controlled depending on the size requirements. Once curing was complete, acetone was used to wash off un-polymerized UV glue. Figure 11(b) shows an image of a lens which is formed using this technique but which does not form part of the invention. Figure 11(c) shows the corresponding ray diagram, which illustrates the quasi-collimated output. Lenses of this type can be used in micro-endoscopic devices for bio-imaging, and in communication to enhance the coupling efficiency.

The microlens tipped fibre and the transfection system of the present invention provide numerous advantages over prior art systems. For example, the microlens tipped fibre can be designed to have a longer working distance (15 - 20 µm) compared to the axicon tipped fibre of the prior art, which makes it easy to position and focus on a cell membrane. In contrast to the axicon tipped fibre based transfection, transfection with a microlensed fibre does not need focusing and re-focusing for transfection of each cell. During the transfection experiments described above, the beam focus was fixed at 5 µm above bottom of the sample petri dish, which was the average height of the cells being investigated. Without any further axial positioning, the tip of the microlensed fibre could be laterally scanned in order to transfect different, individual cells within one Petri dish.

A skilled person will appreciate that variations of the disclosed arrangements are possible without departing from the invention. Whilst the transfection of DNA is described above, it will be appreciated that any material of interest could be introduced into the cell, for example RNA, and various dyes such as Propedium Iodide (PI) and Tryphan Blue. Accordingly the above description of the specific embodiment is made by way of example only and not for the purposes of limitation. It will be clear to the skilled person that minor modifications may be made without significant changes to the operation described.

## Claims

1. A system for transfecting material into a cell comprising a single mode optical fibre (12) that has a microlens formed at its end for directing light to a surface of the cell, and a channel for delivery of the material for transfection into the cell, wherein the microlens comprises a polymerized UV cured adhesive projection extending from a central region of an end facet of the optical fibre, the polymerized UV cured adhesive projection having a curved facet formed at an apex of the polymerized UV cured adhesive projection
wherein formation of the microlens comprises:
dipping the optical fibre in a drop of UV curable adhesive;
raising the optical fibre from the drop of adhesive to form a hemisphere of the adhesive on the end of the optical fibre;
aligning a UV laser beam and the optical fibre relative to one another so that a waist of the UV laser beam is positioned in the optical fibre behind a centre of the end facet of the optical fibre whilst keeping the optical power of the UV laser beam below a threshold optical power required for initiating curing of the adhesive;
increasing the optical power of the UV laser beam above the threshold optical power to cure a portion of the adhesive exposed to the UV laser beam and thereby define a shape of the microlens; and
removing unpolymerized adhesive.

2. A system as claimed in claim 1 comprising at least one laser (32) with an output coupled into the optical fibre (12) for delivery of light to the surface of the cell, thereby to form an opening in the cell to allow material to be transfected, optionally wherein the at least one laser (32) is selected from: a femtosecond laser, nano-second laser, pico-second laser and continuous wave laser.

3. A system as claimed in any of the preceding claims wherein the optical fibre (12) is in the delivery channel, and/or wherein the delivery channel comprises a microcapilliary (14).

4. A system as claimed in any of the preceding claims comprising illuminating means (22) for illuminating the cell.

5. A system as claimed in any of the preceding claims, wherein the lens at the end of the fibre is a focusing lens.

6. A system as claimed in any of the preceding claims provided as an integrated device.

7. A system as claimed in claim 6 wherein an inlet port is provided to allow connection of a fluid delivery supply to the integrated delivery channel.

8. A system as claimed in any of the preceding claims, wherein formation of the microlens comprises aligning the UV laser beam and the optical fibre relative to one another so that the waist of the UV laser beam is positioned in the optical fibre approximately 20 µm behind the centre of the end facet of the optical fibre whilst keeping the optical power of the UV laser beam below the threshold optical power required for initiating curing of the adhesive.

## Patentansprüche

1. System für die Transfektion von Material in eine Zelle, das eine optische Monomode-Faser (12) mit einer an ihrem Ende ausgebildeten Mikrolinse zum Lenken von Licht auf eine Oberfläche der Zelle und einen Kanal zum Zuführen des Materials zur Transfektion in die Zelle umfasst, wobei die Mikrolinse einen polymerisierten UV-gehärteten Klebstoffvorsprung umfasst, der sich von einem zentralen Bereich einer Endfacette der optischen Faser erstreckt, wobei der polymerisierte UV-gehärtete Klebstoffvorsprung eine gekrümmte Facette aufweist, die an einem Scheitelpunkt des polymerisierten UV-gehärteten Klebstoffvorsprungs ausgebildet ist,
wobei die Bildung der Mikrolinse Folgendes umfasst:
Eintauchen der optischen Faser in einen Tropfen eines UV-härtbaren Klebstoffs;
Anheben der optischen Faser aus dem Klebstofftropfen, um eine Halbkugel des Klebstoffs am Ende der optischen Faser zu bilden,
Ausrichten eines UV-Laserstrahls und der optischen Faser relativ zueinander, so dass eine Taille des UV-Laserstrahls in der optischen Faser hinter einer Mitte der Endfacette der optischen Faser positioniert wird, während die optische Leistung des UV-Laserstrahls unter einer optischen Schwellenleistung gehalten wird, die zum Einleiten der Aushärtung des Klebstoffs erforderlich ist,
Erhöhen der optischen Leistung des UV-Laserstrahls über den Schwellenwert der optischen Leistung, um einen Teil des dem UV-Laserstrahl ausgesetzten Klebstoffs auszuhärten und dadurch die Form der Mikrolinse zu definieren; und
Entfernen von nicht polymerisiertem Klebstoff.

2. System nach Anspruch 1, das mindestens einen Laser (32) mit einem Ausgang umfasst, der in die optische Faser (12) gekoppelt ist, um Licht auf die Oberfläche der Zelle zuzuführen, um dadurch eine Öffnung in der Zelle zu bilden, die die Transfektion von Material ermöglicht, wobei der mindestens eine Laser (32) optional ausgewählt ist aus: einem Femtosekundenlaser, einem Nanosekundenlaser, einem Pikosekundenlaser und einem Dauerstrichlaser.

3. System nach einem der vorhergehenden Ansprüche, wobei sich die optische Faser (12) im Zuführungskanal befindet, und/oder wobei der Zuführungskanal eine Mikrokapillare (14) umfasst.

4. System nach einem der vorhergehenden Ansprüche, das ein Beleuchtungsmittel (22) zum Beleuchten der Zelle umfasst.

5. System nach einem der vorhergehenden Ansprüche, wobei die Linse am Ende der Faser eine Fokussierlinse ist.

6. System nach einem der vorhergehenden Ansprüche, das als integrierte Vorrichtung bereitgestellt wird.

7. System nach Anspruch 6, wobei ein Einlassport vorgesehen ist, um den Anschluss einer Fluidversorgung an den integrierten Zuführungskanal zu ermöglichen.

8. System nach einem der vorhergehenden Ansprüche, wobei das Bilden der Mikrolinse das Ausrichten des UV-Laserstrahls und der optischen Faser relativ zueinander umfasst, so dass die Taille des UV-Laserstrahls in der optischen Faser etwa 20 µm hinter der Mitte der Endfacette der optischen Faser positioniert ist, während die optische Leistung des UV-Laserstrahls unter der optischen Schwellenleistung gehalten wird, die zum Einleiten der Aushärtung des Klebstoffs erforderlich ist.

## Revendications

1. Système pour transfecter un matériau dans une cellule, comprenant une fibre optique monomode (16) qui comporte une microlentille formée au niveau de son extrémité pour diriger la lumière vers une surface de la cellule, et un canal pour distribuer le matériau pour la transfection dans la cellule, dans lequel la microlentille comprend une projection d'adhésif polymérisé durci aux UV s'étendant depuis une région centrale d'une facette d'extrémité de la fibre optique, la projection d' adhésif polymérisé durci aux UV comportant une facette courbée formée au niveau d'un apex de la projection d'adhésif polymérisé durci aux UV,
dans lequel la formation de la microlentille comprend :
l'immersion de la fibre optique dans une goutte d'adhésif durcissable aux UV ;
le soulèvement de la fibre optique de la goutte d' adhésif pour former un hémisphère de l'adhésif sur l'extrémité de la fibre optique ;
l'alignement d'un faisceau laser UV et de la fibre optique l'un par rapport à l'autre, de sorte qu'une taille du faisceau laser UV est positionnée dans la fibre optique derrière un centre de la facette d'extrémité de la fibre optique, tout en maintenant la puissance optique du faisceau laser UV au-dessous d'une puissance optique seuil requise pour initialiser le durcissement de l'adhésif ;
l'accroissement de la puissance optique du faisceau laser UV au-dessus de la puissance optique seuil pour durcir une partie de l'adhésif exposé au faisceau laser UV et définir ainsi la forme de la microlentille ; et
le retrait de l'adhésif non polymérisé.

2. Système selon la revendication 1, comprenant au moins un laser (32) avec une sortie couplée dans la fibre otique (12) pour délivrer la lumière vers la surface de la cellule, pour former ainsi une ouverture dans la cellule pour permettre la transfection du matériau, dans lequel l'au moins un laser (32) est optionnellement sélectionné parmi : un laser femtoseconde, un laser nanoseconde, un laser picoseconde et un laser à onde continue.

3. Système selon l'une quelconque des revendications précédentes, dans lequel la fibre optique (12) se trouve dans le canal de distribution, et/ou dans lequel le canal de distribution comprend un micro capillaire (14).

4. Système selon l'une quelconque des revendications précédentes, comprenant un moyen d'éclairage (22) pour éclairer la cellule.

5. Système selon l'une quelconque des revendications précédentes, dans lequel la lentille au niveau de l'extrémité de la fibre est une lentille de focalisation.

6. Système selon l'une quelconque des revendications précédentes, fourni en tant que dispositif intégré.

7. Système selon la revendication 6, dans lequel un orifice d'entrée est fourni pour permettre la connexion d'une alimentation de distribution de fluide au canal de distribution intégré

8. Système selon l'une quelconque des revendications précédents, dans lequel la formation de la microlentille comprend l'alignement du faisceau laser UV et de la fibre optique l'un par rapport à l'autre, de sorte que la taille du faisceau laser UV est positionnée dans la fibre optique, environ 20 µm derrière le centre de la facette d'extrémité de la fibre optique, tout en maintenant la puissance optique du faisceau laser UV au-dessous de la puissance optique seuil requise pour initialiser le durcissement de l'adhésif.
